Europäisches Patentamt

European Patent Office

Office européen des brevets

(11) Numéro de publication : **0 334 777 B1**

(12) **FASCICULE DE BREVET EUROPEEN**

(19)

(45) Date de publication du fascicule du brevet :
**20.05.92 Bulletin 92/21**

(21) Numéro de dépôt : **89420094.8**

(22) Date de dépôt : **16.03.89**

(51) Int. Cl.⁵ : **A61K 9/10,** A61K 7/00,
A61K 7/48

(54) **Microémulsion à usage cosmétique ou pharmaceutique.**

(30) Priorité : **18.03.88 FR 8803839**

(43) Date de publication de la demande :
**27.09.89 Bulletin 89/39**

(45) Mention de la délivrance du brevet :
**20.05.92 Bulletin 92/21**

(84) Etats contractants désignés :
**BE CH DE ES FR GB IT LI NL**

(56) Documents cités :
EP-A- 0 087 062
EP-A- 0 216 557
FR-A- 1 274 354
FR-A- 2 524 897

(73) Titulaire : **GATTEFOSSE S.A.**
**36 chemin de Genas**
**F-69800 Saint Priest (FR)**

(72) Inventeur : **Waginaire, Lucien**
**Le Buclet**
**F-38460 Chamagnieu - Cremieu (FR)**
Inventeur : **Denis, Joel**
**Route de Charly Vourles**
**F-69390 Vernaison (FR)**

(74) Mandataire : **Laurent, Michel et al**
**Cabinet LAURENT et CHARRAS, 20, rue Louis**
**Chirpaz B.P. 32**
**F-69131 Ecully Cedex (FR)**

Il est rappelé que : Dans un délai de neuf mois à compter de la date de publication de la mention de la
délivrance du brevet européen toute personne peut faire opposition au brevet européen délivré, auprès
de l'Office européen des brevets. L'opposition doit être formée par écrit et motivée. Elle n'est réputée
formée qu'après paiement de la taxe d'opposition (Art. 99(1) Convention sur le brevet européen).

## Description

L'invention concerne une nouvelle microémulsion destinée notamment à être utilisée dans l'industrie pharmaceutique ou dans la cosmétologie.

Dans la suite de la description et dans les revendications, par "microémulsion", on désigne une solution micellaire homogène transparente, formée de plusieurs constituants, comprenant généralement deux phases non miscibles, à savoir une phase hydrosoluble et une phase lipidique. En pratique, ces émulsions qui comportent des particules dont les dimensions sont comprises entre cent et cinq cents angströms, sont toujours composées de quatre constituants, à savoir respectivement :

. une phase hydrosoluble, telle que de l'eau, un alcool, un polyol, du glycérol, voire des mélanges de ces composés ;

. une phase huileuse, dite aussi lipidique, telle que huiles minérales, végétales, animales ou synthétiques ;

. au moins un agent tensio-actif ;

. au moins un agent cotensioactif.

Comme on le sait, les "agents tensio-actifs" sont des composés chimiques à caractère hydrophile prononcé, destinés à provoquer la formation de micelles. Un agent tensio-actif utilisé seul permet seulement de former une solution micellaire.

En revanche, les "agents cotensioactifs", dénommés également parfois agents cosurfactants, sont également des composés chimiques, mais à caractère plus hydrophobe, destinés à provoquer la solubilisation mutuelle des phases aqueuses et huileuses dans une microémulsion.

Comme on le sait, les microémulsions nécessitent l'emploi combiné d'agents tensio-actifs et d'agents cotensioactifs.

Les microémulsions ont été largement étudiées pour la récupération du pétrole. Dans ce but, elles contiennent de fortes proportions d'agents tensio-actifs agressifs, inutilisables dans les applications pharmaceutiques ou cosmétiques. Dans les industries pharmaceutiques et cosmétiques, on utilise très largement des émulsions. Toutefois, celles-ci sont composées de particules de dimensions supérieures ou égales à un micromètre. La difficulté réside dans leur formation et surtout dans leurs stabilités thermiques et mécaniques.

L'invention pallie ces inconvénients. Elle vise une microémulsion particulièrement adaptée à l'industrie pharmaceutique ou à la cosmétologie, qui soit compatible avec de telles applications, présente une bonne innocuité tant par voie topique, orale, rectale ou transcutanée, et une stabilité thermodynamique, sans commune mesure avec celle des émulsions traditionnelles.

Cette microémulsion à usage pharmaceutique ou cosmétique, du type comportant une phase hydrosoluble et une phase lipidique, comprenant essentiellement en mélange :

– au moins une phase hydrosoluble,

– au moins une phase lipidique,

– au moins un agent tensio-actif à base de polyéthylèneglycol,

– au moins un agent cotensioactif à base de polyglycérol, **se caractérise** :

– en ce que l'agent tensio-actif est un ester mixte de glycérol et dudit polyethylèneglycol, et représente au moins dix pour cent (10 %) en poids du mélange ;

– en ce que l'agent cotensioactif est un ester dudit polyglycérol et d'acides gras liquides, qui est dans le mélange en proportion inférieure à celle de l'agent tensio-actif, en représente au moins cinq pour cent (5 %) en poids du mélange;

– et enfin, en ce que le composé lipidique présente une balance hydrophile/lipophile d'au plus trois.

En d'autres termes, l'invention consiste à avoir sélectionné des agents tensio-actifs et des agents cotensioactifs particuliers, qui une fois combinés avec une phase hydrosoluble et une phase lipidique forment une microémulsion stable, présentant une excellente innocuité, une bonne tolérance cutanée, une quasi absence de toxicité et aucune action détergente. De la sorte, on peut les utiliser avec succès et sans danger tant dans l'industrie pharmaceutique que dans la cosmétologie. Ainsi, l'invention réside à avoir sélectionné des microémulsions particulières pour l'industrie de la cosmétologie et/ou pharmaceutique, et plus précisément à avoir sélectionné des agents tensio-actifs et agents cotensioactifs spécifiques qui, combinés entre eux, permettront de réaliser des microémulsions présentant une excellente inocuité.

Dans le description et dans les revendications :

– par "composé à action détergente", on désigne les composés chimiques destinés à nettoyer par mouillage, solubilisation ou déplacement des salissures ou des souillures et possédant un pouvoir émulsionnant élevé ;

– par "bonne tolérance cutanée", on entend une absence ou une faible irritation par application sur la peau ou sur les muqueuses ;

– on dit "qu'un composé présente une quasi-absence de toxicité" lorsque sa dose létale (DL50) est très élevée.

– enfin, par "ester mixte de glycérol et de polyéthylèneglycol", on désigne des composés ne présentant que des liaisons esters, à l'exclusion de toutes autres liaisons actives, telles que notamment ethers.

Le plus généralement, l'agent tensio-actif représente au moins quinze pour cent (15 %) en poids du mélange et agent cotensioactif et la proportion d'agent cotensioactif est voisine de la moitié de celle de l'agent tensio-actif.

Avantageusement, en pratique :

– pour que les produits tensio-actifs et/ou cotensioactifs présentent une bonne innocuité, c'est-à-dire une excellente tolérance cutanée, et une quasi-absence de toxicité, ils doivent être préparés avec des matières premières aussi pures que possible et en utilisant des catalyseurs non toxiques et non agressifs et enfin, ils doivent être purifiés ;

– dans l'agent tensio-actif, le polyéthylèneglycol a un poids moléculaire moyen compris entre 100 et 1000 et est de préférence est voisin de 400, ce qui lui confère un caractère non détergent ; comme déjà dit, cet agent tensio-actif doit représenter en poids au moins dix pour cent (10 %) du poids de la microémulsion ; en effet, on a constaté que si ce poids est inférieur à dix pour cent (10 %), on obtient non plus une microémulsion, mais simplement une émulsion traditionnelle, c'est-à-dire opaque, alors que si cette proportion est plus élevée, la dispersion en sera d'autant plus facilitée, mais le coût en sera d'autant plus élevé, sans amélioration proportionnelle ; on obtient de bons résultats avec une proportion d'agent tensio-actif voisin de vingt (20%) ;

– dans l'agent cotensioactif, le polyglycérol a un poids moléculaire compris entre 100 et 1000 et l'acide gras est avantageusement un acide gras liquide, tel que par exemple l'acide oléique ou l'acide isostéarique, seuls ou en mélange ; comme déjà dit, la proportion de cet agent cotensioactif est généralement inférieure à celle de l'agent tensio-actif, mais supérieure à cinq pour cet (5 %), de préférence dix pour cent (10 %) ;

– comme phase lipidique, on utilise tout composé huileux liquide d'usage courant pour les applications pharmaceutiques et/ou cosmétiques, ayant une balance hydrophile/lipophile (HLB) au plus égale à trois, de préférence voisine de deux ;

– la phase hydrosoluble peut être de l'eau ou tout autre composé à caractère hydrosoluble, tels que les alcools, les polyols, le glycerol.., seuls ou en mélange ;

– enfin, la microémulsion peut comporter également :

    . des principes actifs pharmaceutiques que l'on désire introduire ultérieurement en l'état ou en solution dans un solvant approprié ;

    . des agents conservateurs, tels que notamment des agents antiseptiques,

    . des parfums, notamment pour l'application en cosmétologie,

    . des dérivés biologiques ou végétaux,

    . d'autres additifs cosmétiques.

Pour la préparation des émulsions, on a déjà proposé d'utiliser des agents tensio-actifs à base de polyéthylèneglycol ou à base d'ester de polyglycérol et d'acide gras (voir par exemple EPA-0216557 et US-A-4 555 524 et 4 690 774). L'invention ne vise pas ces composés dans leur application comme agents tensio-actifs, mais consiste à avoir déterminé qu'en mélangeant certains de ces composés spécifiques dans des proportions appropriées, à savoir agents tensio-actifs majoritaires mais au moins égal à 10 %, on pouvait préparer des micro-émulsions stables présentant une excellente inoccuité, ce que l'on ne savait faire jusqu'alors, à condition également que la balance hydrophile/lipophile (HLB) de la phase lipidique soit au plus égale à 3 et de préférence voisine de 2.

Le mélange de ces différents composés s'effectue dans des proportions préalablement déterminées par l'expérience, sans précaution particulière ou ordre spécifique, notamment à température ambiante, sous faible agitation.

La manière dont l'invention peut être réalisée et les avantages qui en découlent ressortiront mieux des exemples de réalisation qui suivent, donnés à titre indicatif et non limitatif.

### Exemple 1 :

Dans un becher, on mélange à température ambiante :

– 32,9 grammes d'un agent tensio-actif constitué par un coester en C8/C10 de glycerol et de polyéthylèneglycol de poids moléculaire moyen 400, commercialisé par le Demandeur sous la dénomination "LABRASOL" ;

– 13,2 grammes d'un agent cotensioactif constitué par un méthyl-2 heptadécanoate de polyglycerol commercialisé par le Demandeur sous la dénomination **"PLUROL ISOSTEARIQUE"** ;

– 41,8 grammes d'eau déminéralisée ;

– 12,0 grammes de méthyl-2 heptadécanoate de méthyl-2 heptadécanoyle (composé lipidique) de HLB voisin de 2, commercialisé par le Demandeur sous la dénomination **"ISOSTEARATE D'ISOSTEARYLE"** ;

– 0,1 gramme d'un agent conservateur antiseptique commercialisé par ROHM et HAAS sous la dénomination "KATHON-CG", et constitué par une solution aqueuse de :

    . 5-chloro 2-méthyl 4-isothiazoline 3-one

    . et de 2-méthyl 4-isothiazoline 3-one, complexées par du chlorure de magnésium et stabilisée par du nitrate de magnésium.

A température ambiante, on agite ce mélange à la main pendant cinq minutes environ.

On obtient alors une microémulsion transparente, fluide et limpide, légèrement teintée en jaune, stable dans une gamme de température comprise entre - 20°C et +50°C.

Cette microémulsion convient parfaitement comme excipient pour l'industrie pharmaceutique ou comme base pour la cosmétologie du fait de sa bonne stabilité et de son excellente innocuité cutanée.

**Exemple 2 :**

Dans un bécher, on mélange à température ambiante :
– 32,1 grammes de l'agent tensio-actif **LABRASOL** du Demandeur décrit à l'exemple 1 ;
– 16,1 grammes du même agent cotensioactif **"PLUROL ISOSTEARIQUE"** qu'à l'exemple 1;
– 10,7 grammes du composé lipidique décrit à l'exemple 1, commercialisé par le Demandeur sous la dénomination **"ISOSTEARATE D'ISOSTEARYLE"** ;
– 2,2 grammes d'huile d'amandes douces ;
– 1,1 gramme d'huile de germes de céréales ;
– et enfin 0,5 gramme d'un parfum commercialisé par le Demandeur sous la dénomination **"ROSALEVRE - MA 1445"**.
On ajoute ensuite à ce mélange :
– 26,8 grammes d'eau déminéralisée ;
– 8,0 grammes d'un extrait placentaire aqueux commercialisé par le Demandeur sous la dénomination **"PHYLDERM FILATOV"** ;
– 2,4 grammes d'extrait hydroglycolique **Calendula**, commercialisé le Demandeur sous la dénomination **"VEGETOL MCF 774"** ;
– enfin, 0,1 gramme de l'agent conservateur antiseptique de l'exemple 1.
Après agitation à la main pendant cinq minutes environ à température ambiante, on obtient une microémulsion limpide de couleur jaune orangée, particulièrement adaptée comme préparation cutanée d'hygiène corporelle.

**Exemple 3 :**

Dans 22,5 grammes d'eau déminéralisée, on disperse 5,6 grammes d'un composé commercialisé par le Demandeur sous la dénomination **"ATELOGLYCANE"**, et 0,1 gramme du même agent conservateur antiseptique qu'à l'exemple 1.
On ajoute toujours à température ambiante dans cette dispersion :
– 53,1 grammes de l'agent tensio-actif de l'exemple 1 ;
– 9,7 grammes de l'agent cotensioactif de l'exemple 1 ;
– 9,0 grammes du composé lipidique ISOSTEARATE d'ISOSTEARYLE de l'exemple 1.
On mélange doucement à température ambiante pendant cinq minutes.
On obtient une microémulsion stable, limpide, de couleur jaune, utilisable pour des applications capillaires.

**Exemple 4 :**

Dans 15,5 grammes de propylèneglycol, on mélange :
– 29,9 grammes de l'agent tensio-actif dénommé **LABRASOL** décrit à l'exemple 1,
– 42,5 grammes de l'agent cotensioactif dénommé **"PLUROL ISOSTEARIQUE"**, également décrit à l'exemple 1,
– 12,1 grammes de la phase lipidique **"ISOSTEARATE d'ISOSTEARYLE"**, décrite aussi à l'exemple 1.
On mélange à température ambiante pendant cinq minutes sous faible agitation.
On obtient une microémulsion anhydre, stable, limpide, de couleur jaune, utilisable avec succès comme excipient cosmétologique, notamment pour les soins de la peau.
Les microémulsions préparées conformément à l'invention présentent de nombreux avantages. On peut citer :
– grande innocuité, notamment cutanée, transcutanée, rectale ou orale ;
– excellente stabilité aux températures usuelles d'emploi, c'est-à-dire dans une gamme allant de -30°C à +80°C ;
– absence de séparation en cas de congélation, ce qui facilite la conservation ;
– amélioration de la biodisponibilité des principes actifs pharmaceutiques ;
– possibilité de stérilisation par la chaleur et par filtration stérilisante.
Ces microémulsions peuvent avoir des présentations variées, telles que microémulsions traditionnelles, mousses, formes pressurisées, aérosols, gélules, capsules, ampoules, etc. .
De la sorte, elles peuvent être utilisées avec succès dans toutes les applications cosmétiques ou pharmaceutiques externes cutanées (topiques), internes (orales, rectales, injectables), ou comme supports, vecteurs, excipients à forte compatibilité physiologique.

**Revendications**

1. Microémulsion à usage cosmétique ou pharmaceutique, du type comportant une phase hydrosoluble et une phase lipidique comprenant en mélange :
. au moins une phase hydrosoluble,
. au moins une phase lipidique,
. au moins un agent tensio-actif à base de polyéthylèneglycol,
. au moins un agent cotensioactif à base de polyglycérol,
**caractérisée :**
– en ce que l'agent tensio-actif est un ester mixte de glycérol et dudit polyéthylèneglycol, et représente au moins dix pour cent (10 %) en poids du mélange ;

– en ce que l'agent cotensioactif est un ester dudit polyglycérol et d'un acide gras liquide, qui est dans le mélange en proportion inférieure à celle de l'agent tensio-actif, en représente au moins cinq pour cent (5 %) en poids du mélange ;

– en ce que la phase lipidique présente une balance hydrophile/lipophile d'au plus trois.

2. Microémulsion selon la revendication 1, caractérisée en ce que le polyéthylèneglycol de l'agent tensio-actif a un poids moléculaire moyen voisin de 400.

3. Microémulsion selon la revendication 1, caractérisée en ce que l'agent cotensioactif est un ester d'un acide gras liquide et d'un polyglycerol ayant un poids moléculaire compris entre 100 et 1000.

4. Microémulsion selon la revendication 3, caractérisée en ce que l'acide gras liquide est choisi dans le groupe comprenant l'acide oléique et l'acide isostéarique.

5. Microémulsion selon l'une des revendications 1 à 4, caractérisée en ce que la phase lipidique a une balance hydrophile/lipophile voisine de 2.

6. Microémulsion selon l'une des revendications 1 à 5, caractérisée en ce que la phase hydrosoluble est choisi dans le groupe comprenant l'eau, les alcools, les polyols, le glycerol, seuls ou en mélange.

7. Microémulsion selon l'une des revendications 1 à 6, caractérisée en ce que le mélange comporte également des principes actifs pharmaceutiques, seuls ou en solution dans un solvant.

8. Microémulsion selon l'une des revendications 1 à 7, caractérisée en ce que l'agent tensio-actif représente au moins quinze pour cent (15 %) en poids du mélange, et en ce que la proportion de l'agent cotensioactif est voisine de la moitié de celle de l'agent tensio-actif.

9. Microémulsion selon l'une des revendications 1 à 6, caractérisée en ce que le mélange comporte également des composés choisis dans le groupe constitué par les agents conservateurs, les parfums, les dérivés biologiques ou végétaux, les additifs cosmétiques.


**Patentansprüche**

1. Mikroemulsion, die eine wasserlösliche und eine lipidische Phase aufweist, zur kosmetischen und pharmazeutischen Verwendung, folgendes in Mischung enthaltend:
. zumindest eine wasserlösliche Phase,
. zumindest eine lipidische Phase,
. zumindest ein grenzflächenaktives Agens auf Basis von Polyethylenglykol,
. zumindest ein co-grenzflächenaktives Agens auf Basis von Polyglycerin,

dadurch gekennzeichnet,
– daß das grenzflächenaktive Agens ein gemischter Ester an Glycerin und des genannten Polyethylenglykoles ist, und zumindest zehn Gew.-% der Mischung darstellt;
– daß das co-grenzflächenaktive Agens ein Ester aus dem genannten Polyglycerin und einer flüssigen Fettsäure ist, der in der Mischung in einem Anteil vorhanden ist, der unter dem Anteil des grenzflächenaktiven Agens liegt und zumindest fünf Gew.-% der Mischung darstellt;
– daß die lipidische Phase ein hydrophil/lipophil-Gleichgewicht von höchstens drei aufweist.

2. Mikroemulsion nach Anspruch 1, dadurch gekennzeichnet, daß das Polyethylenglykol des grenzflächenaktiven Agens ein mittleres Molekulargewicht von etwa 400 aufweist.

3. Mikroemulsion nach Anspruch 1, dadurch gekennzeichnet, daß das co-grenzflächenaktive Agens ein Ester aus einer flüssigen Fettsäure und einem Polyglycerin ist, der ein Molekulargewicht zwischen 100 und 1000 aufweist.

4. Mikroemulsion nach Anspruch 3, dadurch gekennzeichnet, daß die flüssige Fettsäure ausgewählt ist aus der Gruppe bestehend aus Ölsäure und Isostearinsäure.

5. Mikroemulsion nach einem der Ansprüche 1 bis 4, dadurch gekennzeichnet, daß die lipidische Phase ein hydrophil/ lipophil-Gleichgewicht von etwa 2 aufweist.

6. Mikroemulsion nach einem der Ansprüche 1 bis 5, dadurch gekennzeichnet, daß dierwasserlösliche Phase ausgewählt ist aus der Gruppe bestehend aus Wasser, Alkoholen, Polyolen, Glycerin, und zwar jeweils die Einzelstoffe oder Mischungen davon.

7. Mikroemulsion nach einem der Ansprüche 1 bis 6, dadurch gekennzeichnet, daß die Mischung außerdem pharmazeutisch aktive Stoffe als solche oder in Lösung in einem Lösungsmittel enthält.

8. Mikroemulsion nach einem der Ansprüche 1 bis 7, dadurch gekennzeichnet, daß das grenzflächenaktive Agens zumindest 15 Gew.-% der Mischung darstellt, und daß der Anteil des co-grenzflächenaktiven Agens etwa die Hälfte des Anteils des grenzflächenaktiven Agens beträgt.

9. Mikroemulsion nach einem der Ansprüche 1 bis 6, dadurch gekennzeichnet, daß die Mischung außerdem Bestandteile ausgewählt aus der Gruppe bestehend aus Konservierungsstoffen, Parfums, biologischen und pflanzlichen Derivaten, kosmetischen Zusatzstoffen enthält.

## Claims

1. A micro-emulsion with pharmaceutical or cosmetic use, of the type comprising a water-soluble phase and a lipidic phase, essentially comprising, in mixture :

. at least one water-soluble phase,

. at least one lipidic phase,

. at least one surface-active agent based on polyethyleneglycol,

. at least one co-surfactant agent based on polyglycerol, is characterized in that :

– the surface-active agent is a mixed ester of glycerol and said polyethyleneglycol, and represents at least ten percent (10%) by weight of the mixture ;

– the co-surfactant agent is an ester of said polyglycerol and liquid fatty acids, which is, in the mixture, in a proportion lower than that of the surface-active agent, and represents at least five per cent (5%) by weight of the mixture ;

– and finally, the lipidic compound presents a hydrophilic/lipophilic balance of three (3) at the most.

2. The micro-emulsion of Claim 1, wherein the polyethyleneglycol of the surface-active agent has an average molecular weight close to 400.

3. The micro-emulsion of Claim 1, wherein the co-surfactant agent is an ester of a liquid fatty acid and a polyglycerol having a molecular weight of between 100 and 1000.

4. The micro-emulsion of Claim 3, wherein the liquid fatty acid is selected from the group comprising oleic acid and isostearic acid.

5. The micro-emulsion of one of Claimsl to 4 wherein the lipidic phase has a hydrophilic/lipophilic balance close to 2.

6. The micro-emulsion of one of Claims 1 to 5, wherein the water-soluble phase is selected from the group comprising water, alcohols, polyols, glycerol, alone or in mixture.

7. The micro-emulsion of one of Claims1 to 6, wherein the mixture also comprises pharmaceutical active ingredients, alone or in solution in a solvent.

8. The micro-emulsion of one of Claims1 to 7, wherein the surface-active agent represents at least fifteen per cent (15%) by weight of the mixture, and the proportion of the co-surfactant agent is close to half that of the surface-active agent.

9. The micro-emulsion of one of Claims1 to 6, wherein the mixture also comprises compounds selected from the group constituted by conservation agents, perfumes, biological or plant derivatives, and cosmetic additives.